(19) Europäisches Patentamt
European Patent Office
Office européen des brevets

(11) Veröffentlichungsnummer: **0 002 779**
**B1**

(12) **EUROPÄISCHE PATENTSCHRIFT**

(21) Anmeldenummer: **78101756.1**

(22) Anmeldetag: **19.12.78**

(51) Int. Cl.³: **C 07 C 67/08,**
**C 07 C 69/14, C 07 C 69/16**

(54) Verfahren zur Vervollständigung der Veresterung von Carbonsäuren mit Alkoholen

(30) Priorität: **20.12.77 DE 2756748**

(43) Veröffentlichungstag der Anmeldung:
**11.07.79 Patentblatt 79/14**

(45) Bekanntmachung des Hinweises auf die Patenterteilung:
**23.07.80 Patentblatt 80/15**

(84) Benannte Vertragsstaaten:
**be DE FR GB IT NL**

(56) Entgegenhaltungen:
**US - A - 2 147 341**
**US - A - 2 645 660**

(73) Patentinhaber: **BASF Aktiengesellschaft**
**Carl-Bosch-Strasse 38**
**D - 6700 Ludwigshafen (DE)**

(72) Erfinder: **Hohenschutz, Heinz, Dr.**
**Leibnizstrasse 12**
**D - 6800 Mannheim 1 (DE)**
**Gnad, Josef, Dr.**
**Londoner Ring 55**
**D - 6700 Ludwigshafen (DE)**
**Dinkhauser, Guenter**
**Tilsiter Strasse 63**
**D - 6703 Limburgerhof (DE)**
**Schaefer, Eberhard**
**Berliner Strasse 21 c**
**D - 6700 Ludwigshafen (DE)**

Verfahren zur Vervollständigung der Veresterung von Carbonsäuren mit Alkoholen

Diese Erfindung betrifft ein neues kontinuierliches Verfahren zur Vervollständigung der Veresterung von Carbonsäuren mit Alkoholen.

Bekanntlich werden Carbonsäureester aus aliphatischen Carbonsäuren und Alkoholen durch Erhitzen der Ausgangsstoffe in Gegenwart von Katalysatoren hergestellt. Bei der großtechnischen Veresterung niederaliphatischer Carbonsäuren wird die Veresterung unter laufender Entfernung des bei der Reaktion gebildeten Wassers durchgeführt. Der dabei erhältliche Rohester wird in der Regel mit wäßrigem Alkali neutralisiert und abschließend einer Feindestillation unterworfen.

Nach einem in Chemie-Ingenieur-Technik 1971, Band 43, Seite 1 001 beschriebenen Verfahren zur kontinuierlichen Herstellung von Carbonsäureestern, setzt man die Ausgangskomponenten in Gegenwart eines Katalysators in einer Veresterungskolonne um, die aus einem Reaktionsteil unterhalb des Zulaufes und einem Verstärkungsteil besteht. Das Wasser wird azeotrop über Kopf abdestilliert. Der Verstärkungsteil hat die Aufgabe, die organische Säure aus dem aufsteigenden Wasser-Azeotrop abzutrennen. Der Rohester wird dampfförmig kurz oberhalb des Sumpfes der Veresterungskolonne abgezogen. Er enthält noch überschüssigen Alkohol, welcher als Leichtsieder in der anschließenden Reinesterkolonne abdestilliert wird.

Da man die Veresterung in Gegenwart saurer Katalysatoren, wie Schwefelsäure oder p-Toluolsulfonsäure durchführt, und bei der Veresterung Wasser gebildet wird, werden bei der großtechnischen Herstellung von Carbonsäureestern an den metallischen Werkstoffen der Reaktionsgefäße, wie Reaktionskesseln oder Kolonnen, Korrosionsschäden beobachtet, die einem ungestörten kontinuierlichen Dauerbetrieb entgegenstehen. Diese Schwierigkeiten treten besonders unter den Bedingungen einer angestrebten vollständigen Umsetzung der Reaktionsteilnehmer auf.

Es bestand daher die Aufgabe, ein Verfahren zu entwickeln, das es gestattet, die Herstellung der Carbonsäureester unter möglichst vollständiger Umsetzung der Reaktionsteilnehmer und weitgehender Zurückdrängung der Korrosion an den Veresterungsapparaten zu erreichen und damit einen störungsfreien Daurbetrieb bei der Carbonsäureesterproduktion zu ermöglichen.

Die vorliegende Erfindung, durch die diese Aufgabe gelöst wird, betrifft ein Verfahren zur Vervollständigung der Veresterung aliphatischer Carbonsäuren, die 1 bis 8 C-Atome aufweisen, mit aliphatischen oder cycloaliphatischen Alkoholen mit 3 bis 10 C-Atomen, durch Behandlung eines Gemisches, das neben dem schon gebildeten Carbonsäureester noch nicht umgesetzte Anteileder Carbonsäure und des Alkohols sowie einen Veresterungskatalysator, Reaktionswasser und gegebenenfalls ein Schleppmittel enthält, bei der Siedetemperatur des Alkohols oder des Schleppmittels in einer mit Sumpfheizung betriebenen Kolonne, wobei das Veresterungswasser durch azeotrope Destillation über den Kopf der Kolonne abgetrieben wird, bei dem der untere Raum der Kolonne frei von Einbauten ist, der mittlere Kolonnenraum mit Füllkörpern und der obere Kolonnenraum mit Füllkörpern oder anderen Einbauten ausgerüstet ist, und bei dem man das Ausgangsgemisch 5 bis 10 m oberhalb des Flüssigkeitsstandes seitlich in die Kolonne einleitet, den gebildeten Ester flüssig dem Kolonnensumpf entnimmt und die Kolonne so geflutet betreibt, daß der Flüssigkeitsstand 6 bis 10 m beträgt, wobei das flüssige Gemisch den von Einbauten freien unteren Kolonnenraum sowie den unteren Teil der darüberliegenden Füllkörperschicht ausfüllt.

Die nach dem neuen Verfahren erhältlichen Ester werden aus Carbonsäuren mit 1 bis 8 C-Atomen und Alkoholen mit 3 bis 10 C-Atomen gebildet. Geeignete Säuren sind z.B. Essigsäure, Propionsäure, Buttersäure oder Äthylhexansäure. Als Alkohole kommen beispielsweise aliphatische oder cycloaliphatische ein- oder mehrwertige Alkohole, wie Propanol, Butanol, Pentanol, Äthylhexanol, Methyl-, Äthyl-, Butylglykol oder p-tert.-Butylcyclohexanol in Betracht. Diese Ausgangsstoffe werden in der Regel in stöchiometrischer Menge verwendet. Man kann aber auch den Alkohol bzw. die Säure in einem Überschuß bis zu 50% zur stöchiometrischen Menge einsetzen.

Als Katalysatoren verwendet man an sich bekannte Veresterungskatalysatoren, z.B. nicht oxidierende Mineralsäuren, wie Schwefelsäure, Salzsäure, Phosphorsäure oder organische Sulforsäuren wie Benzosulfonsäure, Methansulfonsäure oder p-Toluolsulfonsäure.

Schleppmittel verwendet man üblicherweise nur dann, wenn der gebildete Rohester oder der gegebenenfalls im Überschuß eingesetzte Alkohol keine oder nur ungenügende wasserschleppende Eigenschaften besitzt. Geeignete Schleppmittel sind z.B. Isobutylacetat, N-Butylacetat, Benzol, Chlorbenzol, Dichloräthan und Butanol.

Nach dem erfindungsgemäßen Verfahren wird die Veresterung der genannten Ausgangsstoffe vervollständigt, d.h. es wird von einem Gemisch ausgegangen, das aus den Ausgangsstoffen in einer üblichen Veresterungsapparatur, wie Rührkessel, Reaktionsblase oder Verweilzeitbehälter bei Temperaturen von 50 bis 200°C erhalten wurden. Dieses Gemisch enthält z.B. 10 bis 30 Gewichtsprozent der Carbonsäure, 10 bis 30 Gewichtsprozent des Alkohols, 50 bis 80 Gewichtsprozent des Carbonsäureesters aus der genannten Carbonsäure und dem genannten Alkohol, 2 bis 15 Gewichtsprozent Wasser, 0,05 bis 3 Gewichtsprozent eines Veresterungskatalysators und gegebenenfalls ein Schleppmittel.

Die vollständige Veresterung wird in einer Kolonne vorgenommen, die mit Sumpfheizung betrieben wird und die im unteren Kolonnenraum frei von Einbauten ist. Dieser untere Kollonnenraum nimmt von der Höhe der Kolonne, die z.B. 20 bis 28 m beträgt, die unteren 4 bis 6 m ein. Der mittlere Kolonnenraum ist mit Füllkörpern beschickt. Der untere Teil dieser Füllkörperschicht ist von dem flüssigen Raktionsgemisch bedeckt. Der obere Teil der Füllkörperschicht, der vom oberen Flüssigkeitsstand bis zur Höhe des Zulaufes des Ausgangsgemisches reicht (Abtriebsteil), hat eine Bodenzahl von etwa 7 bis 11. Der obere Kolonnenraum, vom Zulauf des Ausgangsgemisches gerechnet (Verstärkungsteil), der ebenfalls mit Füllkörpern oder mit anderen Einbauten wie Glocken-Ventil- oder Siebböden ausgerüstet ist, hat eine Bodenzahl von etwa 9 bis 14.

Der innere Durchmesser der Kolonnen beträgt etwa 80 bis 120 cm.

Das vorveresterte Gemisch wird nach dem neuen Verfahren zweckmäßig bei der Siedetemperatur des Alkohols oder des Schleppmittels und bei Normaldruck, erhöhtem oder erniedrigtem Druck, vorzugsweise aber bei Normaldruck von der Seite her und oberhalb des Flüssigkeitsstandes in den mittleren Kolonnenraum eingeleitet. Wasser und Schleppmittel werden über den Kopf der Kolonne abgetrieben. Der gebildete Rohester wird dem flüssigen Gemisch am Sumpf der Kolonne entnommen. Durch Steuerung des Rohesterablaufes hält man den Flüssigkeitsstand in der Kolonne auf 6 bis 10 m. Dabei soll das flüssige Gemisch den von Einbauten freien unteren Kolonnenraum vollständig und die darüberliegende Füllkörperschicht zum Teil ausfüllen. Zweckmäßig werden 1 bis 6, vorzugsweise 3 bis 5 m, der Füllkörperschicht von der Flüssigkeit bedeckt.

Nach dem erfindungsgemäßen Verfahren läßt sich die Carbonsäureesterherstellung im technischen Maßstab kontinuierlich durchführen, wobei auch bei jahrelangem Betrieb nur geringfügige Korrosionserscheinungen beobachtet werden. Diese vorteilhafte Wirkung hat vermutlich ihre Ursache darin, daß der Wassergehalt im flüssigen Roestergemisch in der Kolonne beim erfindungsgemäßen Verfahren nicht über 0,05 Gewichtsprozent liegt. Da sich ein derart vorteilhafter niedriger Wassergehalt nicht einstellt, wenn der untere Teil der Kolonne nicht geflutet wird, sondern der Flüssigkeitsstand in der Kolonne, z.B. entsprechend dem in Chem. Ing. Techn. 1971, Seite 1 001 Beschriebenen Verfahren unterhalb des oberen Rohrbodens des Umlaufverdampfers gehalten wird oder wenn der Flüssigkeitsstand auf andere beleibige Höhe eingestellt wird, Muß das nach dem neuen Verfahren erhaltene günstige Ergebnis als überraschend bezeichnet werden.

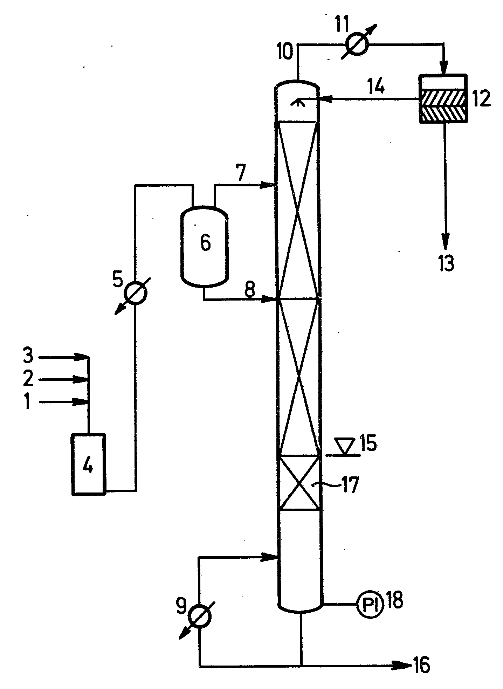

Das Verfahren nach der Erfindung führt man, wie in der Abbildung veranschaulicht, z.B. in einer Kolonne mit einer Höhe von 24 m und einem Durchmesser von 90 cm durch. Der untere Kolonnenraum ist bis einer Höhe von 500 cm frei von Einbauten. Der darüberliegende Reaktionsraum ist mit Füllkörpern beschickt. Die seitliche Zuleitung für das Veresterungsgemisch befindet sich 15 m oberhalb des unteren Kolonnenendes. Der Kolonnenteil unterhalb der Zuleitung (Abtreibsteil) hat eine Bodenzahl von 10, der Kolonnenteil oberhalb der Zuleitung (Verstärkungsteil) hat eine Bodenzahl von 11.

Butanol (1), Essigsäure (2) und Schwefelsäure (3) werden kontinuierlich in einen Gemischbehälter (4) eingeleitet. Das über einen Wärmeaustauscher (5) vorgewärmte Gemisch der Ausgangsstoffe wird in einem der Kolonne vorgeschalteten Reaktionsraum (6) bei etwa 90°C vorverestert, wobei ein Teil des entstandenen Veresterungswassers dampfförmig in den oberen Teil der Kolonne eingeführt wird (7). Das vorveresterte Gemisch wird durch die Zuleitung (8) in die Veresterungskolonne geleitet, die durch einen Naturumlaufverdampfer (9) beheizt wird. Die Temperatur in der Kolonne beträgt etwa 110°C.

Am Kopf der Kolonne wird über die Leitung (10) ein Azeotrop aus Wasser, Alkohol und Ester entnommen und kondensiert (11). Das in der Vorlage (12) anfallende Destillat bildet zwei Phasen. Die wäßrige untere Phase wird über die Leitung (13) zur weiteren Aufarbeitung abgeführt, die organische obere Phase wird über die Leitung (14) also Rücklauf auf den Kopf der Kolonne geleitet. Der Flüssigkeitsstand (15) im Sumpf der Reaktionskolonne wird über die Rohesterablaufmenge (16) auf einer Höhe von 6 bis 10 m gehalten. Der obere Flüssigkeitsstand ragt dabei 400 cm in die untere Füllkörperschicht (17) hinein. Die Kontrolle des Flüssigkeitsstandes in der Kolonne erfolgt Zweckmäßig durch Messung des statischen Druckes PI (18) am unteren Kolonnenende. Auch Druckdifferenz-Messungen zwischen Kopf- und Sumpfteil der Veresterungskolonne sind moglich.

Der aus der Kolonne abgezogene Rohester wird in bekannter Weise weiterverarbeitet, d.h. neutralisiert, gewaschen und rektifiziert.

### Beispiel 1

In die in der Abbildung dargestellt und oben beschriebene Kolonne leitet man ein Gemisch, das man aus 2 165 Teilen/h Essigsäure, 3 135 Teilen/h n-Butanol und 64 Teilen/h Schwefelsäure durch Vermischen und Erwärmen auf 90°C erhalten hat. Es enthält 60 Gewichtsprozent Essigsäurebutylester, 18 Gewichtsprozent Essigsäure, 14 Gewichtsprozent n-Butanol, 6,8 Gewichtsprozent Wasser und 1,2 Gewichtsprozent Schwefelsäure.

Das Veresterungswasser wird über den Kopf der Kolonne kontinuierlich ausgekreist. Die Sumpftemperatur beträgt 143°C. Im Kopf der Kolonne stellt sich eine Temperatur von 92°C ein. Durch Einstellen eines statischen Druckes von 1,63 bar im Sumpf hält man den Flüssigkeitsstand in der Kolonne auf 850 cm. Der obere Flüssigkeitsstand ragt dabei 400 cm in die untere Füllkörperschicht hinein. Der aus der Kolonne abgezogene Rohester hat einen Wassergehalt von 0,031% und eine Säurezahl von 10,6 mg KOH/g Substanz.

### Beispiel 2

Entsprechend Beispiel 1 werden pro Stunde 1 487 Teile Essigsäure, 2, 158 Teile iso-Butanol und 46,8 Teile Schwefelsäure in die Vorveresterung gegeben. Das Veresterungsgemisch, das indie Kolonne geleitet wird, enthält 60 Gewichtsprozent Essigsäureisobutylester, 15 Gewichtsprozent iso-Butanol, 18 Gewichtsprozent Essigsäure, 5,7 Gewichtsprozent Wasser und 1,3 Gewichtsprozent Schwefelsäure. Die Sumpftemperatur beträgt 133°C. Im Kopf der Kolonne stellt sich eine Temperatur von 90°C ein. Durch Einstellen eines statischen Druckes von 1,65 bar im Sumpf wird in der Kolonne ein Flüssigkeitsstand von 8,80 m eingehalten. Der abgezogene Rohester hat einen Wassergehalt von 0,05 Gewichtsprozent und eine Säurezahl von 10,2 mg KOH/g Substanz.

### Beispiel 3

Entsprechend Beispiel 1 werden pro Stunde 696 Teile Essigsäure, 1 184 Teile Äthylenglykolmonoäther, 783 Teile/h iso-Butylacetat (als Schleppmittel) und 17,1 Teile/h Schwefelsäure über die Vorveresterung in die Kolonne geleitet. Das vorveresterte Gemisch enthält 43,8 Gewichtsprozent Essigsäureglykolester, 13,3 Gewichtsprozent Äthylenglykolmonoäther, 10,1 Gewichtsprozent Essigsäure, 29,2 Gewichtsprozent Essigsäureisobutylester, 3,0 Gewichtsprozent Wasser und 0,6 Gewichtsprozent Schwefelsäure.

Die Sumpftemperatur beträgt 142°C. Im Kolonnenkopf stellt sich eine Temperatur von 88°C ein. Durch Einstellen eines statischen Druckes von 1,8 bar im Sumpf stellt sich ein Flüssigkeitsstand von 8,0 m ein. Das Veresterungswasser wird mit dem Schleppmittel kontinuierlich über den Kolonnenkopf ausgekreist. Der erhaltene Rohester hat einen Wassergehalt von 0,035 Gewichtsprozent und die Säurezahl von 12 mg KOH/g Substanz.

### Patentansprüche

1. Kontinuierliches Verfahren zur Vervollständigung der Veresterung aliphatischer Carbonsäuren, die 1 bis 8 C-Atome aufweisen, mit aliphatischen oder cycloaliphatischen Alkoholen mit 3 bis 10 C-Atomen, durch Behanlung eines Gemisches, das neben dem schon gebildeten Carbonsäureester noch nicht umgesetzte Anteile der Carbonsäure und des Alkohols sowie einen Veresterungskatalysator, Reaktionswasser und gegebenenfalls ein Schleppmittel enthält, bei der Siedetemperatur des Alkohols oder des Schleppmittels in einer mit Sumpfheizung betriebenen Kolonne, wobei das Veresterungswasser durch azeotrope Destillation über den Kopf der Kolonne abgetrieben wird, *dadurch gekennzeichnet,* daß der untere Raum der Kolonne frei von Einbauten ist, der mittlere Kollonnenraum mit Füllkörpern und der obere Kolonnenraum mit Füllkörpern oder anderen Einbauten ausgerüstet ist, daß man das Ausgangsgemisch 5 bis 10 m oberhalb des Flüssigkeitsstandes seitlich in die Kolonne einleitet, den gebildeten Ester flüssig dem Kolonnensumpf entnimmt und die Kolonne so geflutet betreibt, daß der Flüssigkeitsstand 6 bis 10 m beträgt, wobei das flüssige Gemisch den von Einbauten freien unteren Kolonnenraum sowie den unteren Teil der darüberliegenden Füllkörperschicht ausfüllt.

2. Verfahren nach Anspruch 1, *dadurch gekennzeichnet,* daß das Ausgangsgemisch 10 bis 30 Gewichtsprozent der Carbonsäure, 10 bis 30 Gewichtsprozent des Alkohols, 50 bis 80 Gewichtsprozent des Esters der Carbonsäure und des Alkohols, 2 bis 15 Gewichtsprozent Wasser, 0,05 bis 3 Gewichtsprozent eines Veresterungskatalysators und gegebenenfalls ein Schleppmittel enthält.

3. Verfahren nach Anspruch 1, *dadurch gekennzeichnet,* daß die Höhe der Kolonne 20 bis 28 m, der innere Durchmesser der Kolonne 80 bis 120 cm und der untere von Einbauten freie Kolonnenraum 4 bis 6 m beträgt, der obere Teil der Füllkörperschicht, der vom oberen Flüssigkeitsstand bis zur Höhe des Zulaufs des Ausgangsgemisches reicht, eine Bodenzahl von 7 bis 11 und der obere Kolonnenraum von Zulauf des Ausgangsgemisches gerechnet, eine Bodenzahl von 9 bis 14 aufweist.

4. Verfahren nach Anspruch 1, *dadurch gekennzeichnet,* daß in der Kolonne 1 bis 6 m der Füllkörperschicht über dem von Einbauten freien unteren Kolonnenraum von dem flüssigen Gemisch ausgefüllt wird.

### Revendications

1. Procédé mené en continu pour compléter l'estérification d'acides carboxyliques aliphatiques qui comportent 1 à 8 atomes de carbone, avec des alcools aliphatiques ou cycloaliphatiques contenant 3 à 10 atomes de carbone, par traitement d'un mélange qui contient, outre l'ester d'acide carboxylique déjà formé, des fractions n'ayant pas réagi de l'acide carboxylique et de l'alcool, ainsi qu'un catalyseur d'estérification et, le cas échéant, un produit d'entraînement, à la température d'ébullition de l'alcool ou du produit d'entraînement dans une colonne exploitée avec chauffage du fond, l'eau d'estérification étant chassée par distillation azéotropique par le sommet de la colonne, caractérisé en ce que la partie inférieure de la colonne est dépourvue d'éléments incorporés, sa partie moyenne est chargée d'éléments de garnissage et sa partie supérieure est munie d'éléments de garnissage ou d'autres éléments incorporés, et en ce qu'on introduit le mélange de départ latéralement dans la colonne à une hauteur de 5 à 10 m au-dessus du niveau de liquide, on prélève l'ester formé à l'état liquide au fond de la colonne et on maintient la colonne suffisament inondée pour que le niveau de liquide se situe entre 6 et 10 m, le mélange liquide remplissant complètement la partie inférieure de la colonne dépourvue d'éléments incorporés, ainsi que la partie inférieure de la couche susjacente d'éléments de garnissage.

2. Procédé selon la revendication 1, caractérisé en ce que le mélange de départ contient 10 à 30% en poids de l'acide carboxylique, 10 à 30% en poids de l'alcool, 50 à 80% en poids de l'ester de l'acide carboxylique et de l'alcool, 2 à 15% en poids d'eau, 0,05 à 3% en poids d'un catalyseur d'estérification et, le cas échéant, un produit d'entraînement.

3. Procédé selon la revendication . 1, caractérisé en ce que la hauteur de la colonne mesure 20 à 28 m, le diamètre intérieur de la colonne 80 à 120 cm et la partie inférieure de la colonne dépourvue d'éléments incorporés 4 à 6 m, et en ce que la partie supérieure de la couche d'éléments de garnissage, qui s'étend du niveau supérieur du liquide jusqu'à la hauteur de l'admission du mélange de départ, présente un nombre de plateaux de 7 à 11 et la zone supérieure de la colonne, calculée à partir de l'admission du mélange de départ, présente un nombre de plateaux de 9 à 14.

4. Procédé selon la revendication 1, caractérisé en ce que dans la colonne, 1 à 6 m de la couche d'éléments de garnissage audessus de la partie inférieure de la colonne dépourvue d'éléments incorporés sont remplis complètement du mélange liquide.

**Claims**

1. A continuous process for completing the esterification of aliphatic carboxylic acids of 1 to 8 carbon atoms with aliphatic or cycloaliphatic alcohols of 3 to 10 carbon atoms by treating a mixture which in addition to the carboxylic acid ester already formed contains as yet unconverted proportions of carboxylic acid and alcohol, together with an esterifying catalyst and water of reaction, and with or without an entraining agent, at the boiling point of the alcohol or of the entraining agent, in a column operated with bottom heating, the water of esterification being driven off at the top of the column by azeotropic distillation, *characterized in that* the lower space of the column is free from fitments, the middle space of the column is provided with a packing and the upper space of the column is provided with a packing or other fitments, the starting mixture is fed into the side of the column from 5 to 10 m above the liquid level, the ester formed is taken off as liquid from the column bottom, and the column is operated with flooding such that the liquid level is from 6 to 10 m, with the liquid mixture filling the lower column space, which is free from fitments, and the lower part of the layer of packing above the said space.

2. A process as claimed in claim 1, *characterized in that* the starting mixture contains from 10 to 30 per cent by weight of carboxylic acid, from 10 to 30 per cent by weight of the alcohol, from 50 to 80 per cent by weight of the ester of the carboxylic acid and the alcohol, from 2 to 15 per cent by weight of water and from 0.05 to 3 per cent by weight of an esterifying catalyst, with or without an entraining agent.

3. A process as claimed in claim 1, *characterized in that* the height of the column is 20 to 28 m, the internal diameter of the column is 80 to 120 cm, and the lower space of the column which is free from fitments, has a height of 4 to 6 m, the upper part of the layer of packing, which extends from the upper liquid level to the height of the feed point of the starting mixture, comprises 7 to 11 plates and the upper space of the column, from the feed point of the' starting mixture upwards, comprises 9 to 14 plates.

4. A process as claimed in claim 1, *characterised in that* 1 to 6 m of the packed layer in the column, above the lower column space which is free from fitments, is filled by the liquid mixture.